# EUROPEAN PATENT SPECIFICATION

(11) **EP 3 696 549 B1**
(45) Date of publication and mention of the grant of the patent: **03.07.2024**
(21) Application number: 18866891.7
(22) Date of filing: 18.07.2018
(51) Int. Cl.: G01N 33/49, G01N 35/00

(54) **AUTOMATED ANALYZER**
AUTOMATISIERTER ANALYSATOR
ANALYSEUR AUTOMATIQUE

(30) Priority: 10.10.2017 JP 2017196811
(43) Date of publication of application: 19.08.2020
(73) Proprietor: Hitachi High-Tech Corporation, Minato-ku Tokyo 105-6409 (JP)
(72) Inventor: MATSUOKA, Yuya, Tokyo 105-8717 (JP); MAKINO, Akihisa, Tokyo 105-8717 (JP)
(74) Representative: MERH-IP Matias Erny Reichl Hoffmann Patentanwälte PartG mbB
(86) International application number: PCT/JP2018/026800
(87) International publication number: WO 2019/073649

(56) References cited:
- EP-A1- 3 514 548
- WO-A1-2016/006362
- JP-A- H 085 562
- JP-A- H11 352 131
- JP-A- 2005 207 767
- JP-A- 2014 119 329
- JP-A- 2014 119 329
- JP-B2- 4 949 389
- US-A- 5 590 052

## Description

### Technical Field

The present disclosure relates to an automated analyzer that automatically analyzes a component included in a biological sample such as blood or urine.

### Background Art

As an apparatus that analyzes a component included in a sample such as blood, an automated analyzer that measures the amount of scattered light or transmitted light having a single or multiple wavelengths generated when light is irradiated from a light source to a reaction solution, which is an analysis target, as a mixture of a sample and a reagent is known.

Examples of the automated analyzer include an apparatus for biochemical analysis that performs qualitative or quantitative analysis of a target component in a biological sample in the field of a biological examination or a hematological examination and an apparatus for blood clotting analysis that measures clotting ability of blood as a sample.

In particular, regarding a clotting time analysis item such as PT, APTT, or fibrinogen that measures clotting ability of blood, a specimen and a reagent are actually mixed with each other to clot the specimen, and the progress of the clotting reaction is measured using an optical or physical method to examine the clotting time. In the measurement of the clotting time item, the specimen is clotted in the reaction vessel. Therefore, the reaction vessel is disposed. In order to analyze the clotting time, not only an analysis unit, a sample dispensing mechanism, and a reagent dispensing mechanism but also a reaction vessel transfer mechanism that transfers a reaction vessel to a position where a specimen or a reagent is dispensed, an analysis unit, and a disposing unit are necessary.

As an analysis flow of an automated blood clotting analyzer, for example, a flow in which a vessel into which a specimen and a reagent are previously dispensed is transferred to an analysis unit, a flow in which a vessel into which only a specimen is dispensed is transferred to an analysis unit and subsequently a reagent is dispensed into the vessel, or a flow in which an empty vessel is transferred to an analysis unit and subsequently a specimen and a reagent are dispensed into the vessel is known.

In the automated analyzer that measures the above-described clotting reaction, a disposable reaction vessel is used. Therefore, whether or not a reaction vessel is placed in a detector may be detected. PTL 1 discloses an automated analyzer in which a reaction vessel presence-absence detection function also serves as a light detection function of a detector. PTL 2 disclosed an automatic analysis device with an optical configuration in which, if a reactor vessel is not installed in a measurement port, part of the light from the light source enters the detection unit, and if a reactor vessel is installed in a measurement port, then light from the light source does not enter the detection unit. PTL 3 describes an automatic analysis apparatus with an analysis port comprising a reaction container holding part, a light source, and a detector.

### Citation List

### Patent Literature

PTL 1: JP 2014 119329 A
PTL 2: WO 2016/006362 A1
PTL 3: EP 3 514 548 A1

### Summary of Invention

### Technical Problem

In a clinical examination, the accuracy of an analysis result and the reliability of an apparatus are required. In particular, the automated blood clotting analyzer is used for a case such as pre-operation where emergency is required in many cases. Therefore, it is required that a highly reliable analysis result can be immediately reported. In particular, in the case of an urgent specimen, the time from specimen loading to result reporting is extremely important, and it is necessary to minimize delay of result reporting caused by problems of the apparatus.

In addition, a clotting reaction starts immediately after mixing a specimen and a reagent with each other and ends in a short period of time such as several seconds. Therefore, it is necessary that the measurement of the clotting reaction starts immediately after mixing a specimen and a reagent with each other and continues at a short sampling interval. Further, the accuracy of temperature control affects the reaction rate, which affects the accuracy or reproducibility of clotting time analysis. Therefore, it is desirable that a reaction solution is kept at a temperature very close to 37°C from when a specimen and a reagent are mixed with each other.

Regarding the above-described two points, when an analysis flow in which a heated specimen and a heated reagent are dispensed using a probe into a reaction vessel that is previously set on an analysis unit and heated or an analysis flow in which a heated reagent is dispensed using a probe after setting a reaction vessel into which a specimen is previously dispensed on an analysis unit and heating the reaction vessel is adopted, the measurement can start immediately after mixing the specimen and the reagent with each other, and by controlling the temperatures of only the analysis unit and the probe, the measurement can start in a state where the temperature of a reaction solution is stable. Therefore, it can be said that the analysis flow is preferable as compared to an analysis flow in which a reaction solution is heated after mixing a specimen and a reagent with each other.

However, in the above-described dispensing method, when the setting of the reaction vessel fails due to the occurrence of a defect in the reaction vessel transfer mechanism and the failure cannot be detected, a specimen or a reagent is discharged into an analysis unit containing an optical component or an electronic component, which may bring about the risk of a serious malfunction of the apparatus. In order to reduce the risk, addition of a sensor that detects the setting of a reaction vessel on an analysis unit may be considered. However, the manufacturing costs increase.

On the other hand, in the case of a method in which a reaction vessel into which a specimen and a reagent are previously dispensed is set on an analysis unit using a transfer mechanism, there is no risk that the specimen or the reagent is discharged into the analysis unit containing an optical component or an electronic component. However, in this method, the measurement cannot be performed during a period from the mixing of the specimen and the reagent to completion of the setting of the reaction vessel. Therefore, the reliability of the measurement result deteriorates.

Separately from the above-described method of detecting the setting of the reaction vessel, regarding an automated blood clotting analyzer that performs clotting time analysis by irradiating the reaction vessel with light from below and detecting light that is scattered to the side, PTL 1 discloses a technique of detecting the setting of the reaction vessel on the analysis unit using two methods including: a method of detecting light scattered from a specimen; and a method of detecting light reflected from a bottom surface of the spherical reaction vessel.

However, in the method described in PTL 1, an empty reaction vessel cannot be detected in the analysis unit adopting the method of irradiating the reaction vessel with light from the side and detecting light that is scattered to the side. Therefore, the method of detecting the reaction vessel described in PTL 1 cannot be widely applied to the above-described various analysis flows. In addition, in the method described in PTL 1, for detecting light reflected from the bottom surface, it is necessary to continue photometry at a short sampling interval. Therefore, a restriction of a circuit and software or the number of processes increases, which may cause an increase in costs.

The present disclosure has been made in consideration of the above-described points and provides a technique capable of measuring a clotting reaction time with high accuracy and detecting a placement state of a reaction vessel at a low cost.

### Solution to Problem

In order to achieve the above-described object, the invention is set out in the appended set of claims.

### Advantageous Effects of Invention

According to the present disclosure, a clotting reaction time can be measured with high accuracy, and a placement state of a reaction vessel can be detected at a low cost. Objects, configurations, and effects other than those described above will be clarified by describing the following embodiment.

### Brief Description of Drawings

[Fig. 1] Fig. 1 is a diagram illustrating a basic configuration of an automated analyzer according to Embodiment 1.
[Fig. 2] Fig. 2 is a diagram illustrating a basic configuration of a blood clotting time detecting unit.
[Fig. 3] Fig. 3 is a diagram illustrating an optical configuration of the blood clotting time detecting unit.
[Fig. 4] Fig. 4 is a flowchart illustrating an example of a placement check operation of a reaction vessel (for blood clotting analysis).
[Fig. 5] Fig. 5 is a diagram illustrating a state of detecting whether or not a reaction vessel (for blood clotting analysis) into which a specimen, a diluent, or the like is dispensed is placed in an analysis port.
[Fig. 6] Fig. 6 is a diagram illustrating an example of an optical configuration of a blood clotting time detecting unit according to Embodiment 2.
[Fig. 7] Fig. 7 is a diagram illustrating an example of an optical configuration of a blood clotting time detecting unit according to Embodiment 2.

### Description of Embodiments

Hereinafter, embodiments of the present disclosure will be described based on the drawings . Embodiments of the present disclosure are not limited to the embodiments described below, and various modifications can be made within a range of technical thoughts thereof. In addition, corresponding components in the respective drawings used for the description of the respective embodiments described below are represented by the same reference numerals, and the description thereof will not be repeated. Automated analyzers according to Embodiments 1 to 6 have a common hardware configuration. An automated analyzer according to Embodiment 7 is different from the automated analyzers according to Embodiments 1 to 6 in that a recessed transmitted light absorbing hole is provided opposite to a light source with respect to an analysis port. In addition, an automated analyzer according to Embodiment 8 is different from the automated analyzers according to Embodiments 1 to 6, in the position of the light source. It is noted that embodiments 1 to 3, 5 and 7 to 8 are not part of the invention but represent useful examples for understanding the invention.

### <Embodiment 1>

### [Basic Configuration of Apparatus]

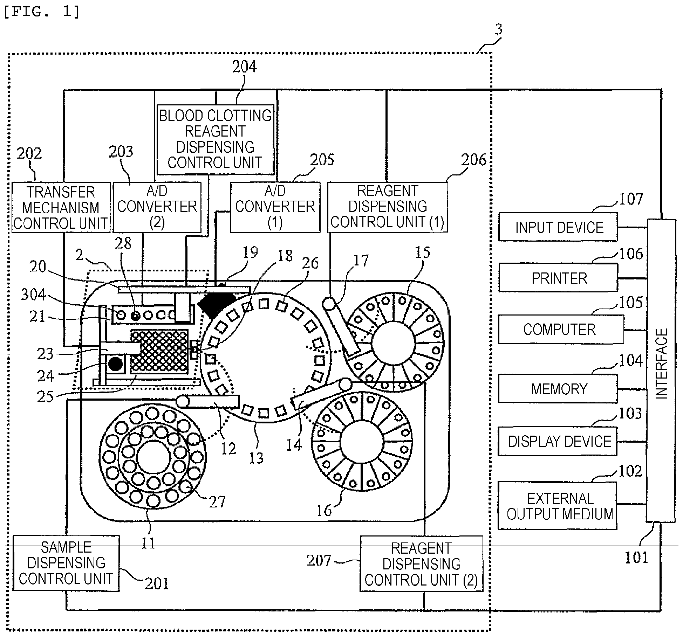

Fig. 1 is a diagram illustrating a basic configuration of an automated analyzer according to Embodiment 1. Here, as an aspect of the automatic analyzer, a composite type automated analyzer 1 including a blood clotting time analyzing unit 2 and a turntable type biochemical analyzing unit 3 will be described.

As illustrated in the drawing, in the automated analyzer 1, a reaction disk 13, a sample disk 11, a first reagent disk 15, a second reagent disk 16, a blood clotting time analyzing unit 2, and a photometer 19 are provided on a housing.

The reaction disk 13 is a disk-shaped unit that is rotatable clockwise and counterclockwise, in which plural reaction vessels (for biochemical analysis) 26 can be arranged on a circumference thereof.

The sample disk 11 is a disk-shaped unit that is rotatable clockwise and counterclockwise, in which plural sample vessels 27 each of which stores a sample such as a standard sample or a test sample can be arranged on a circumference thereof.

The first reagent disk 15 and the second reagent disk 16 are disk-shaped units that are rotatable clockwise and counterclockwise, in which plural reagent vessels each of which stores a reagent can be arranged on a circumference thereof. The above-described reagent contains a component that is reactive with a component of each examination item included in the sample. In addition, the first reagent disk 15 and the second reagent disk 16 may include a cooling mechanism or the like such that the reagent in the arranged reagent vessel can be cooled.

A sample dispensing probe 12 is arranged between the sample disk 11 and the reaction disk 13. By rotating the sample dispensing probe 12, an operation of aspirating and dispensing the sample can be performed on the sample vessel 27 on the sample disk 11, the reaction vessel (for biochemical analysis) 26 on the reaction disk 13, and a reaction vessel (for blood clotting analysis) 28 at a sample dispensing position 18 of the blood clotting time analyzing unit 2.

Likewise, a first reagent dispensing probe 17 is arranged between the first reagent disk 15 and the reaction disk 13, and a second reagent dispensing probe 14 is arranged between the second reagent disk 16 and the reaction disk 13. By rotating each of the first reagent dispensing probe 17 and the second reagent dispensing probe 14, the operation of aspirating and dispensing can be performed on the reaction vessel (for biochemical analysis) 26 on the reaction disk 13 and the reagent vessels on the first reagent disk 15 and the second reagent disk 16.

The blood clotting time analyzing unit 2 mainly includes a blood clotting time detecting unit 21, a blood clotting reagent dispensing probe 20, a reaction vessel magazine 25, a reaction vessel transfer mechanism 23, and a reaction vessel disposal hole 24.

Next, a control system and a signal processing system relating to the automated analyzer 1 will be simply described. A computer 105 is connected to a sample dispensing control unit 201, a transfer mechanism control unit 202, an A/D converter (2) 203, a blood clotting reagent dispensing control unit 204, an A/D converter (1) 205, a reagent dispensing control unit (1) 206, and a reagent dispensing control unit (2) 207 via an interface 101, and a signal as an instruction is transmitted to each of the controllers.

The sample dispensing control unit 201 controls the sample dispensing operation of the sample dispensing probe 12 based on an instruction received from the computer 105.

In addition, the reagent dispensing control unit (1) 206 and the reagent dispensing control unit (2) 207 control the reagent dispensing operation of the first reagent dispensing probe 17 and the second reagent dispensing probe 14 based on an instruction received from the computer 105.

The transfer mechanism control unit 202 controls the operation of the reaction vessel transfer mechanism 23 transferring the disposable reaction vessel (for blood clotting analysis) 28 between the reaction vessel magazine 25, the sample dispensing position 18, an analysis port 304 of the blood clotting time detecting unit 21, and the reaction vessel disposal hole 24 based on an instruction received from the computer 105.

The blood clotting reagent dispensing control unit 204 operates the blood clotting reagent dispensing probe 20 based on an instruction received from the computer 105 such that the reagent for blood clotting is dispensed into the reaction vessel (for blood clotting analysis) 28 that is placed in the analysis port 304 and stores the sample.

In addition, the blood clotting reagent dispensing control unit 204 operates the blood clotting reagent dispensing probe 20 such that a pre-treatment solution as a mixed solution of the sample and a first reagent for blood clotting analysis in the reaction vessel (for biochemical analysis) 26 is dispensed into the empty reaction vessel (for blood clotting analysis) 28. In this case, subsequently, the blood clotting reagent dispensing control unit 204 dispenses a second reagent for blood clotting analysis into the reaction vessel (for blood clotting analysis) 28 storing the pre-treatment solution. The reagents for blood clotting analysis are arranged in the first reagent disk 15 and the second reagent disk 16 and are used for blood clotting analysis after being temporarily dispensed into the reaction vessel (for biochemical analysis) 26 on the reaction disk 13 by the first reagent dispensing probe 17 and the second reagent dispensing probe 14 as necessary.

The A/D converter (1) 205 converts a photometric value of light transmitted through a reaction solution inside the reaction vessel (for biochemical analysis) 26 or light scattered in the reaction solution into a digital signal. In addition, the A/D converter (2) 203 converts a photometric value of light transmitted through a reaction solution inside the disposable reaction vessel (for blood clotting analysis) 28 or light scattered in the reaction solution into a digital signal. The above-described digital signal is input to the computer 105.

A printer 106 for printing a measurement result as a report or the like, a memory 104 or an external output medium 102 as a storage device, an input device 107 such as a keyboard for inputting an operation instruction or the like, and a display device 103 for displaying a screen are connected to the interface 101. Examples of the display device 103 include a liquid crystal display and a CRT display.

### [Analysis Procedure of Biochemical Analytical Item]

Analysis of a biochemical analytical item by the automated analyzer 1 is performed according to the following procedure. First, an operator inputs an examination item for each sample using the input device 107 such as a keyboard. In order to analyze the sample for the input examination item, the sample dispensing probe 12 dispenses a predetermined amount of the sample from the sample vessel 27 into the reaction vessel (for biochemical analysis) 26 according to analysis parameters.

The reaction vessel (for biochemical analysis) 26 into which the sample is dispensed is transferred by the rotation of the reaction disk 13 and is stopped at a reagent receiving position. A pipette nozzles of each of the first reagent dispensing probe 17 and the second reagent dispensing probe 14 dispenses a predetermined amount of reagent solution into the reaction vessel (for biochemical analysis) 26 according to analysis parameters of the corresponding examination item. Regarding the dispensing order of the sample and the reagent, the reagent may be dispensed before the sample contrary to the example.

Next, the sample and the reagent are stirred and mixed using a stirring mechanism (not illustrated). When the reaction vessel (for biochemical analysis) 26 passes through a photometric position, the photometer 19 performs photometry of transmitted light or scattered light of the reaction solution as the mixed solution of the sample and the reagent stored in the reaction vessel (for biochemical analysis) 26. The transmitted light or the scattered light having undergone photometry is converted into numeric data in proportion to the amount of light by the A/D converter (1) 205, and the numeric data is input to the computer 105 via the interface 101.

Component concentration data is calculated based on a calibration curve measured in advance using the converted numeric data with an analysis method designated per examination item. The component concentration data as the analysis result of each examination item is output to the printer 106 or the screen of the display device 103.

Before performing the above-described measuring operation, the operator sets various parameters required for analysis or registers the reagent and the sample through an operation screen of the display device 103. In addition, after the measurement, the operator checks the analysis result through the operation screen on the display device 103.

### [Analysis Procedure of Blood Clotting Time Item]

In addition, analysis of a blood clotting time item by the automated analyzer 1 is performed according to the following procedure.

First, the computer 105 receives the input of an examination item for each sample using the input device 107 such as a keyboard. Next, in order to analyze the sample for the requested examination item, the reaction vessel transfer mechanism 23 transfers the disposable reaction vessel (for blood clotting analysis) 28 from the reaction vessel magazine 25 to the sample dispensing position 18. The sample dispensing probe 12 dispenses a predetermined amount of the sample from the sample vessel 27 into the reaction vessel (for blood clotting analysis) 28 according to analysis parameters.

The reaction vessel (for blood clotting analysis) 28 into which the sample is dispensed is transferred to the analysis port 304 of the blood clotting time detecting unit 21 by the reaction vessel transfer mechanism 23 and is heated to a predetermined temperature.

The first reagent dispensing probe 17 dispenses a predetermined amount of reagent solution into the reaction vessel (for biochemical analysis) 26 on the reaction disk 13 according to analysis parameters of the corresponding examination item. In the reaction disk 13, a thermostat (not illustrated) is provided. Therefore, the reagent solution dispensed into the reaction vessel (biochemical analysis) 26 is heated to 37°C.

Next, the blood clotting reagent dispensing probe 20 aspirates the reagent dispensed into the reaction vessel (for biochemical analysis) 26, the reagent is heated to a predetermined temperature by a heating mechanism (not illustrated) in the blood coagulation reagent dispensing probe 20, and the heated reagent is discharged into the reaction vessel (for blood clotting analysis) 28.

When the reagent is discharged, photometry of transmitted light or scattered light of light irradiated to the reaction vessel (for blood clotting analysis) 28 starts. The transmitted light or the scattered light having undergone photometry is converted into numeric data in proportion to the amount of light by the A/D converter (2) 203, and the numeric data is input to the computer 105 via the interface 101.

The computer 105 obtains the time required for the blood clotting reaction (hereinafter, also simply referred to as "blood clotting time") using the converted numerical value. For example, regarding an examination item such as ATPP (activated partial thromboplastin time), the computer 105 outputs the blood clotting time obtained as described above as the analysis result. On the other hand, regarding an examination item such as Fbg (fibrinogen), the computer 105 further obtains component concentration data with respect to the obtained blood clotting time and output the obtained component concentration data as the analysis result based on a calibration curve measured in advance using the converted numerical value with an analysis method designated per examination item. The blood clotting time or the component concentration data as the analysis result of each examination item is output to the printer 106 or the screen of the display device 103.

Here, before performing the above-described measuring operation, the operator sets various parameters required for analysis or registers the reagent and the sample in advance through the operation screen of the display device 103. The computer 105 analyzes each examination item based on the registered various parameters and the analysis procedure. In addition, after the measurement, the operator can check the analysis result through the operation screen on the display device 103.

In addition, the place into which the sample is discharged by the sample dispensing probe 12 may be the reaction vessel (for biochemical analysis) 26. In this case, after causing the sample to react with the pre-treatment solution in advance in the reaction vessel (for biochemical analysis) 26, the sample can also be dispensed into the reaction vessel (for blood clotting analysis) 28 by the blood clotting reagent dispensing probe 20.

In the reaction vessel (for blood clotting analysis) 28, stirring called discharge stirring is performed on the sample stored in the reaction vessel (for blood clotting analysis) 28 in advance. Here, the discharge stirring refers to a method of mixing the reagent with the sample in the reaction vessel (for blood clotting analysis) 28 using a force with which the reagent is discharged. Regarding the dispensing order of the sample and the reagent, the reagent may be dispensed before the sample contrary to the example. In this case, the sample can be mixed with the reagent using a force with which the sample is discharged.

### [Structure of Blood Clotting Time Detecting Unit]

Next, the blood clotting time detecting unit 21 will be described. The blood clotting time detecting unit 21 includes a light source 302 and a detector 303 in one or plural analysis ports 304 where the disposable reaction vessel (for blood clotting analysis) 28 can be placed, and the blood coagulation time detecting unit 21 can detect an intensity of scattered light of light irradiated to the reaction solution in the reaction vessel (for blood clotting analysis) 28 or an intensity of light reflected from the surface of the above-described analysis port.

The blood clotting reagent dispensing probe 20 performs the aspiration operation of the reagent stored in the reaction vessel (for biochemical analysis) 26 on the reaction disk 13 and the dispensing operation of the reagent into the reaction vessel (for blood clotting analysis) 28 placed in the blood clotting time detecting unit 21.

The reaction vessel magazine 25 is used for aligning and placing plural disposable reaction vessels (for blood clotting analysis) 28. The sample dispensing position 18 is provided for dispensing the sample as the analysis target placed in the sample disk 11 into the reaction vessel (for blood clotting analysis) 28. Here, the reaction vessel (for blood clotting analysis) 28 is transferred from the reaction vessel magazine 25 by the reaction vessel transfer mechanism 23.

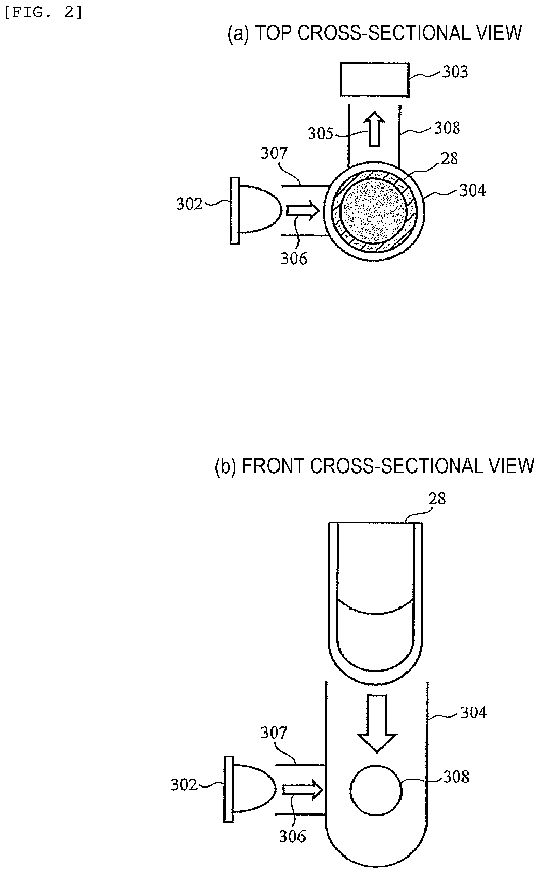

Fig. 2 is a diagram illustrating a basic configuration of the blood clotting time detecting unit 21. Specifically, Fig. 2 illustrates an arrangement configuration of the reaction vessel (for blood clotting analysis) 28, the light source 302, and the detector 303 in the analysis port 304.

(a) of Fig. 2 is a top cross-sectional view illustrating the arrangement configuration of the light source 302 and the detector 303 when seen from the top. (b) of Fig. 2 is a front cross-sectional view illustrating the arrangement configuration when seen from the front. (b) of Fig. 2 illustrates a state where the reaction vessel (for blood clotting analysis) 28 is not yet placed in the analysis port 304.

The blood clotting time detecting unit 21 includes light source 302 and the detector 303 as described above and further includes one or plural analysis ports 304 where the disposable reaction vessel (for blood clotting analysis) 28 can be provided from the top.

The detector 303 is arranged on a side surface of the reaction vessel (for blood clotting analysis) 28 such that scattered light 305 of light (irradiated light 306) irradiated from the light source 302 to the reaction solution in the reaction vessel (for blood clotting analysis) 28 can be detected. An irradiation slit 307 that adjusts an irradiation range of the irradiated light 306 is provided between the light source 302 and the reaction vessel (for blood clotting analysis) 28, and a light receiving slit 308 that adjusts a range of the scattered light 305 is provided between the reaction vessel (for blood clotting analysis) 28 and the detector 303.

### [Method of Detecting Reaction Vessel]

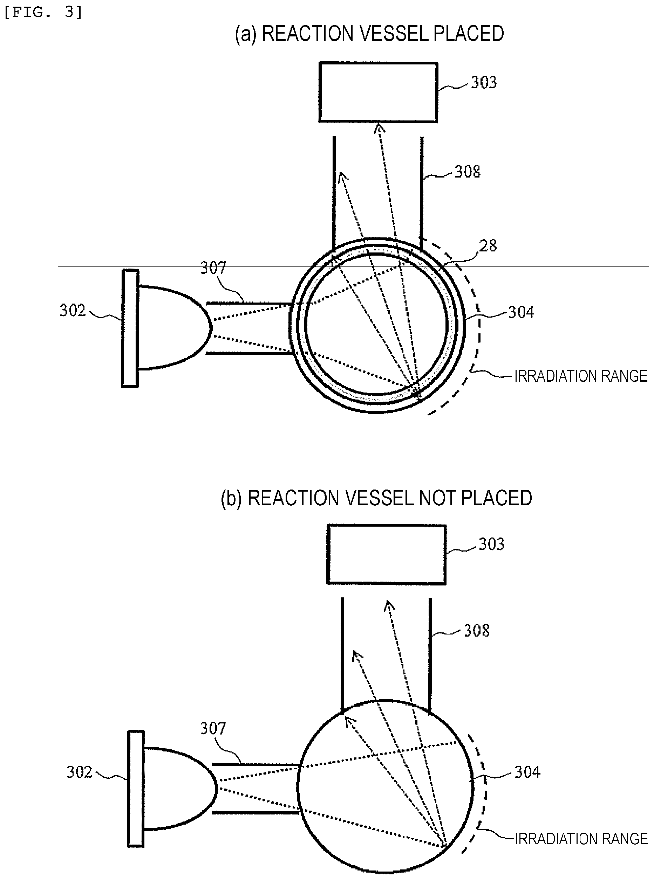

Fig. 3 is a diagram illustrating an optical configuration of the blood clotting time detecting unit 21.
(a) of Fig. 3 illustrates a path of light when the empty reaction vessel (for blood clotting analysis) 28 is placed in the analysis port 304. The light irradiated from the light source 302 collides with an inner wall surface of the analysis port 304 that is surface-treated such that the light is refracted and slightly reflected when incident on the reaction vessel (for blood clotting analysis) 28 and when emitted from the reaction vessel (for blood clotting analysis) 28. Most of the light that collides with the wall surface is absorbed, but a part of the light is diffused and reflected to be incident on the detector 303.
(b) of Fig. 3 illustrates light rays when the reaction vessel (for blood clotting analysis) 28 is not placed in the analysis port 304. As illustrated in the drawing, when the reaction vessel (for blood clotting analysis) 28 is not placed, the above-described refraction of the light does not occur. Therefore, a range where the light irradiated from the light source 302 collides with the wall surface of the analysis port 304 is narrower than that when the reaction vessel (for blood clotting analysis) 28 is placed. At this time, a part of the light that is diffused and reflected from the wall surface of the analysis port 304 is incident on the detector 303. In this case, the amount of light detected by the detector 303 is less than that when the empty reaction vessel (for blood clotting analysis) 28 is placed.

Accordingly, by comparing an output of the detector 303 in a state where the reaction vessel (for blood clotting analysis) 28 is not placed in the analysis port 304 and an output of the detector 303 in a state where the empty reaction vessel (for blood clotting analysis) 28 is placed to each other, whether or not the empty reaction vessel (for blood clotting analysis) 28 is placed in the analysis port 304 can be determined.

Specifically, when a difference between the amount of light measured in a case where the empty reaction vessel (for blood clotting analysis) 28 is placed and the amount of light measured in a case where the empty reaction vessel (for blood clotting analysis) 28 is not placed is more than or equal to a standard value, the computer 105 determines that the reaction vessel (for blood clotting analysis) 28 is normally placed. On the other hand, when the difference is less than the standard value, the computer 105 determines that the placement of the reaction vessel (for blood clotting analysis) 28 fails. Here, the standard value refers to, for example, a value that is appropriately determined by experiment according to the degree of the surface treatment of the analysis port 304. In addition, when the amount of light after the placement of the reaction vessel (for blood clotting analysis) 28 is less than the amount of light before the placement of the reaction vessel (for blood clotting analysis) 28, the computer 105 may determine that the reaction vessel (for blood clotting analysis) 28 fails to be normally placed.

As the reason why the placement of the reaction vessel (for blood clotting analysis) 28 fails, for example, misalignment of the position of the mechanism that transfers the reaction vessel (for blood clotting analysis) 28 or deterioration in alignment accuracy caused by malfunction of the reaction vessel transfer mechanism 23 may be considered. As described above, the amount of light detected by the detector 303 when the placement of the reaction vessel (for blood clotting analysis) 28 fails is not more than the amount of light detected by the detector 303 when the reaction vessel (for blood clotting analysis) 28 is not placed in the analysis port 304.

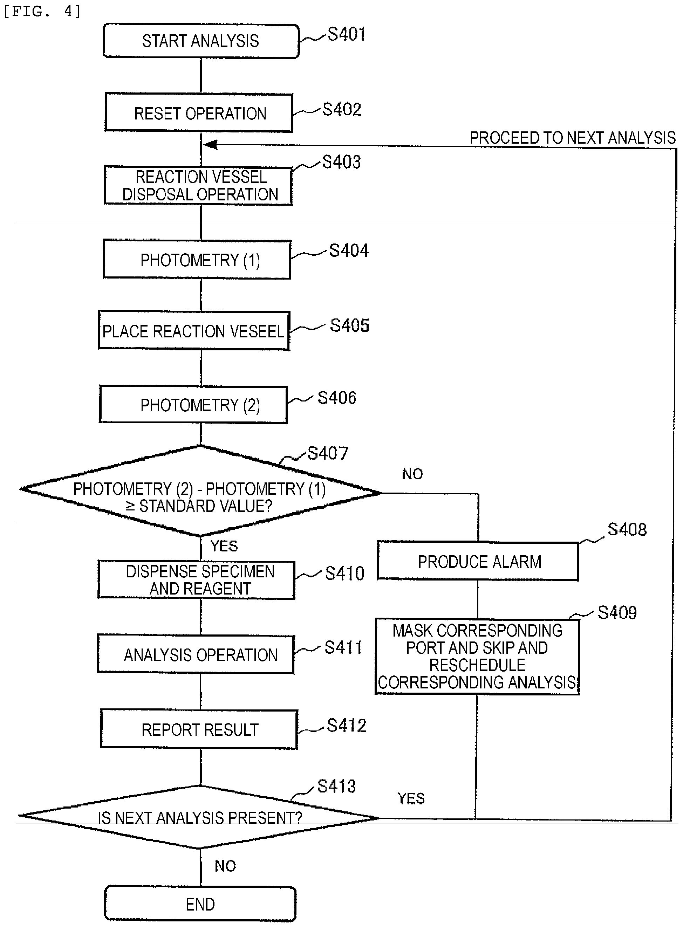

Fig. 4 is a flowchart illustrating an example of a placement check operation of the reaction vessel (for blood clotting analysis) 28. Using Fig. 4, a light amount check operation in the above-described configuration and a method of masking the analysis port based on the light amount check operation will be described.

First, the input device 107 in the automated analyzer 1 receives an input that instructs to start an analysis operation (S401). Next, the automated analyzer 1 performs an operation of resetting the respective mechanisms (S402) and then performs an operation of disposing the reaction vessel (for blood clotting analysis) 28 used for the previous analysis (S403).

Next, in a state where the light source 302 is turned on and the reaction vessel (for blood clotting analysis) 28 is not placed in the analysis port 304, the computer 105 performs photometry using the detector 303 (S404). Here, the light that is received by the detector 303 is the light that is diffused and reflected from the wall surface of the analysis port 304.

Next, the reaction vessel transfer mechanism 23 places the empty reaction vessel (for blood clotting analysis) 28 in the analysis port 304 (S405), and the detector 303 performs photometry (S406). Here, the light that is received by the detector 303 is the light that transmits through the reaction vessel (for blood clotting analysis) 28 and is diffused and reflected from the wall surface of the analysis port 304. When the difference between the amounts of light measured before and after the placement of the reaction vessel (for blood clotting analysis) 28 is more than or equal to the standard value as a result of the photometry (YES in S407), the specimen and the reagent are dispensed into the reaction vessel (for blood clotting analysis) 28 using the corresponding analysis port 304 (S410), and the analysis operation is performed on the reaction solution that is the mixed solution of the sample and the reagent dispensed into the reaction vessel (for blood clotting analysis) 28 (S411). After the analysis ends, the computer 105 reports the result (S412) and determines whether or not the next analysis is present (S413). Here, when the next analysis is present (YES in S413), the process returns to Step S403, and the steps after the reaction vessel disposal operation (S403) are repeated. When the next analysis is not present (NO in S413), the computer 105 ends the operation of placing the reaction vessel (for blood clotting analysis) 28.

On the other hand, when the difference between the amount of light is less than the standard value in Step S407 (NO in S407), as described above, the placement failure of the reaction vessel (for blood clotting analysis) 28 may occur. Therefore, the computer 105 produces a warning (for example, an alarm sound or a screen display) for notification to the operator (S408) and performs a control assuming misalignment of one analysis port 304 such that the analysis port 304 is masked (S409), the use thereof is prevented, and the operations after Step S403 are performed using another analysis port. That is, the reaction vessel transfer mechanism 23 places the reaction vessel (for blood clotting analysis) 28 in an analysis port other than the masked analysis port 304, and the computer 105 performs the operations after Step S403.

Through the above-described operations, whether or not the reaction vessel (for blood clotting analysis) 28 succeeds to be normally placed can be determined. Therefore, the risk of malfunction and emergency maintenance caused by the discharge of the specimen or the reagent into the analysis port 304 or the risk of reporting delay due to reexamination caused by analysis failure can be reduced.

### <Embodiment 2>

In Embodiment 1 described above, when the difference between the amounts of light before and after the placement of the reaction vessel (for blood clotting analysis) 28 is less than the standard value or when the amount of light after the placement is less than the amount of light before the placement, the computer 105 determines that the reaction vessel (for blood clotting analysis) 28 fails to be normally placed.

However, even in a case where the reaction vessel (for blood clotting analysis) 28 is normally placed in the analysis port 304, for example, when light is blocked due to the presence of scratch or contamination of the reaction vessel (for blood clotting analysis) 28 or when light is blocked due to infiltration of foreign matter into the reaction vessel (for blood clotting analysis) 28, the intensity of light detected by the detector 303 changes.

Accordingly, when the difference between the amounts of light before and after the placement of the reaction vessel (for blood clotting analysis) 28 is less than the standard value or when the amount of light after the placement is less than the amount of light before the placement, the computer 105 may determine that the reaction vessel (for blood clotting analysis) 28 cannot be used. Here, a standard value based on which the computer 105 determines that the reaction vessel (for blood clotting analysis) 28 is not normally placed in the analysis port 304 and a standard value based on which the computer 105 determines that the reaction vessel (for blood clotting analysis) 28 is contaminated, may be the same as or different from each other. These standard values are appropriately determined by experiment and appropriately reflect the status.

When the difference between the amounts of light before and after the placement of the reaction vessel (for blood clotting analysis) 28 is less than the standard value based on which the computer 105 determines that the reaction vessel (for blood clotting analysis) 28 is contaminated, the computer 105 has a function of producing a warning for notification to the operator, skipping the specimen dispensing operation, the reagent dispensing operation, the analysis operation, and the result reporting to prevent the use of the reaction vessel (for blood clotting analysis) 28, and rescheduling the analysis to proceed to the next analysis operation. As a result, erroneous reporting of the report caused by an abnormality of the reaction vessel (for blood clotting analysis) 28 can be prevented, the delay of reporting due to analysis failure caused by automatic rescheduling can be minimized, and the reliability of the examination can be improved.

### <Embodiment 3>

In the description of the Embodiment 1, the computer 105 determines whether or not the reaction vessel (for blood clotting analysis) 28 is normally placed in the analysis port 304 by measuring the difference between the amounts of light before and after the placement of the empty reaction vessel (for blood clotting analysis) 28 in the analysis port 304.

However, as described above, in addition to the empty reaction vessel (for blood clotting analysis) 28, the reaction vessel transfer mechanism 23 may also place the reaction vessel (for blood clotting analysis) 28 into which the specimen or the diluent is dispensed in the analysis port 304. That is, the reaction vessel (for blood clotting analysis) 28 may contain liquid in an amount less than a final total amount of the reaction solution.

In this case, even when the specimen, the diluent, or the like is dispensed into the reaction vessel (for blood clotting analysis) 28, as long as the difference between the amounts of light before and after the placement of the reaction vessel (for blood clotting analysis) 28 is more than or equal to the standard value, the computer 105 may determine that the reaction vessel (for blood clotting analysis) 28 is normally placed.

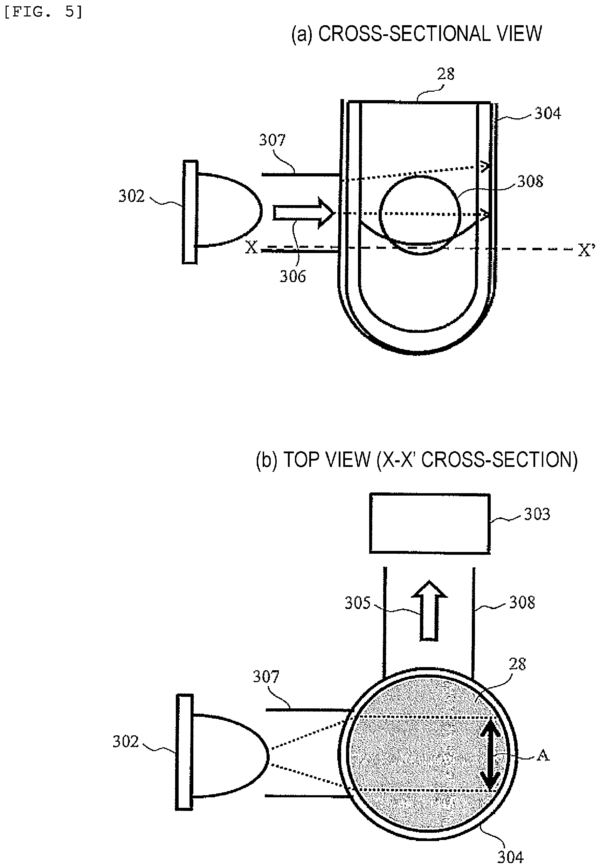

Fig. 5 is a diagram illustrating a state of detecting whether or not the reaction vessel (for blood clotting analysis) 28 into which the specimen, the diluent, or the like is dispensed is placed in the analysis port 304. As illustrated in (a) of Fig. 5, when the irradiation slit 307 and the light receiving slit 308 are placed at a height above the liquid level at which the specimen or the diluent is dispensed, as in (a) of Fig. 3, the light irradiated to the liquid level collides with a wide range of the inner wall surface of the analysis port 304 such that the light that is diffused and reflected from the wall surface is incident on the detector 303. In other words, a space above the liquid level of the liquid is irradiated with at least some of the light emitted from the light source 302.

On the other hand, light that is irradiated below the liquid level is refracted in the reaction vessel and is irradiated to a narrow range of the wall surface as illustrated in (b) of Fig. 5. Here, the refractive index of the reaction vessel (for blood clotting analysis) 28 and the refractive index of the dispensed liquid are values close to each other. Therefore, light is not refracted much on an interface between the reaction vessel (for blood clotting analysis) 28 and the liquid. Accordingly, by appropriately setting the position of the detector 303, even when the light that is irradiated below the liquid level is diffused and reflected, the light is not incident on the detector 303 much.

Here, the surface treatment of the wall surface of the analysis port 304 is controlled such that the amount of light that is incident on the detector 303 through the space above the liquid level is more than the amount of light incident on the detector 303 when the reaction vessel (for blood clotting analysis) 28 is not placed. As a result, even when the liquid does not allow transmission of light at all, whether or not the reaction vessel (for blood clotting analysis) 28 is normally placed can be determined by comparing the differences between the amounts of light before and after the placement of the reaction vessel (for blood clotting analysis) 28. In addition, when the specimen or the diluent is actually dispensed, a larger amount of light than that when liquid having a transmittance of 0 is dispensed is incident on the detector 303. In this case, whether or not the reaction vessel (for blood clotting analysis) 28 is normally placed can be determined by comparing the differences between the amounts of light before and after the placement of the reaction vessel (for blood clotting analysis) 28.

When the specimen or the diluent is dispensed into the reaction vessel (for blood clotting analysis) 28, the scattering intensity of light is different from that when the reaction vessel (for blood clotting analysis) 28 is empty. The standard value of the difference between the amounts of light may be set to be different from the standard value described in the description of Embodiment 1. As a result, whether or not the reaction vessel (for blood clotting analysis) 28 is normally placed can be determined without modifying the surface treatment of the wall surface of the analysis port 304. That is, whether or not the reaction vessel (for blood clotting analysis) 28 is normally placed can be determined by changing the numerical value on software without changing hardware.

### <Embodiment 4>

In the description of Embodiment 1, whether or not the reaction vessel (for blood clotting analysis) 28 is normally placed in the analysis port 304 is determined by comparing the amounts of light before and after the placement of the reaction vessel (for blood clotting analysis) 28. In this method, the determination can be performed without being affected by an individual difference or deterioration of the analysis port 304, the light source 302, and the like. However, by performing calibration on each analysis port 304 in advance, even when the measurement (S404 in Fig. 4) of the amount of light before the placement of the reaction vessel (for blood clotting analysis) 28 is not performed, the determination can be performed without being affected by an individual difference of the component.

Specifically, the amounts of light of three cases are measured and stored in advance, the three cases including: a case where the reaction vessel (for blood clotting analysis) 28 is not placed; a case where the empty reaction vessel (for blood clotting analysis) 28 is placed; and a case where the reaction vessel (for blood clotting analysis) 28 into which the specimen or the diluent (or liquid that does not allow transmission of light) is dispensed is placed. The computer 105 compares a numerical range of each of the three amounts of light having a predetermined width to the value of the amount of light detected after the placement of the reaction vessel (for blood clotting analysis) 28, determines whether the state of the analysis port 304 is in the first case, the second case, or the third case, and produces a warning when the state of the analysis port 304 is different from a predetermined state. Here, the predetermined value refers to, for example, the maximum value of the width of the amount of light that can change when the kind of the specimen and the amount of the specimen dispensed. In addition, "the predetermined state" refers to, for example, a state that should be set with reference to the process procedure registered in the computer 105.

For example, based on whether or not the predetermined operation flow is an operation flow in which the empty reaction vessel (for blood clotting analysis) 28 is placed or an operation flow in which the reaction vessel (for blood clotting analysis) 28 into which the specimen or the diluent is dispensed is placed, the computer 105 determines that the reaction vessel (for blood clotting analysis) 28 fails to be normally placed, determines that the reaction vessel (for blood clotting analysis) 28 should be empty but contains some liquid, or determines the amount of light is less than any of those of the three patterns and the reaction vessel (for blood clotting analysis) 28 contains foreign matter, and outputs a signal for skipping the analysis operation on the reaction vessel (for blood clotting analysis) 28.

In the above-described method, the photometry before the placement of the reaction vessel (for blood clotting analysis) 28 can be removed, the operation can be simplified, and the individual difference of the component can be corrected. Therefore, the reliability of the determination can be improved.

### <Embodiment 5>

In the method according to Embodiment 2, when the amount of light detected after the placement of the reaction vessel (for blood clotting analysis) 28 into which the specimen or the specimen and the diluent are dispensed is more than the amount of light detected when the reaction vessel (for blood clotting analysis) 28 into which the normal specimen is dispensed is normally placed by a predetermined value, the specimen dispensed into the reaction vessel (for blood clotting analysis) 28 is likely to be a specimen having a high turbidity called a chyle specimen. In the above-described case, the computer 105 may add an alarm to the output of the analysis result for notification to the operator.

When the chyle specimen is dispensed into the reaction vessel (for blood clotting analysis) 28, the chyle specimen has high turbidity, and thus the amount of a noise component increases in the measurement of scattered light. Therefore, scattering in the process of the reaction or attenuation of the change in amount of light occurs, and the reliability of the analysis result deteriorates. Accordingly, by notifying this point to the operator, the operator is urged to scan the analysis result. Therefore, the reliability of the examination can be improved.

A threshold of the amount of light for determination that the specimen dispensed into the reaction vessel (for blood clotting analysis) 28 is the chyle specimen can be determined by storing the amount of light when the normal specimen is dispensed in advance in combination of the calibration described in Embodiment 3. As a result, the accuracy of the determination can be improved.

### <Embodiment 6>

In the above-described methods of Embodiments 1, 3, and 4, the computer 105 compares the amount of light after the placement of the reaction vessel (for blood clotting analysis) 28 and the amount of light after the reagent dispensing to each other, and when the amount of light after the placement of the reaction vessel (for blood clotting analysis) 28 is less than or equal to the amount of light after the reagent dispensing, the computer 105 determines that the reagent is normally dispensed and adds an alarm to the analysis result such that this point can be notified to the operator. In other words, when the amount of light that is measured before discharging the reagent into the reaction vessel (for blood clotting analysis) 28 is less than or equal to the amount of light that is measured after discharging the reagent into the reaction vessel (for blood clotting analysis) 28, the computer 105 can produce a warning to the operator together with the output of the analysis result.

At this time, the surface of the analysis port 304 is configured to reflect at least some of light irradiated from the light source 302 in a state where the reaction vessel (for blood clotting analysis) 28 into which the specimen or the specimen and the diluent are dispensed is placed. When all the irradiated light is designed to pass through a space below the liquid level after the reagent dispensing, before the reagent dispensing, the light that passes through the space above the liquid level is diffused and reflected from the wall surface of the analysis port 304 to be incident on the detector 303 as illustrated in (a) of Fig. 5. In this case, after the reagent dispensing, all the irradiated light is refracted and irradiated to a narrow range as illustrated in (b) of Fig. 5. Therefore, the amount of light incident on the detector 303 decreases, and only scattered light is present in the reaction solution.

As a result, by additionally determining whether or not the reagent is normally dispensed, not only the operation check of the reaction vessel transfer mechanism 23 but also the operation check of the reagent dispensing mechanism can be performed, and the reliability can be improved.

### <Embodiment 7>

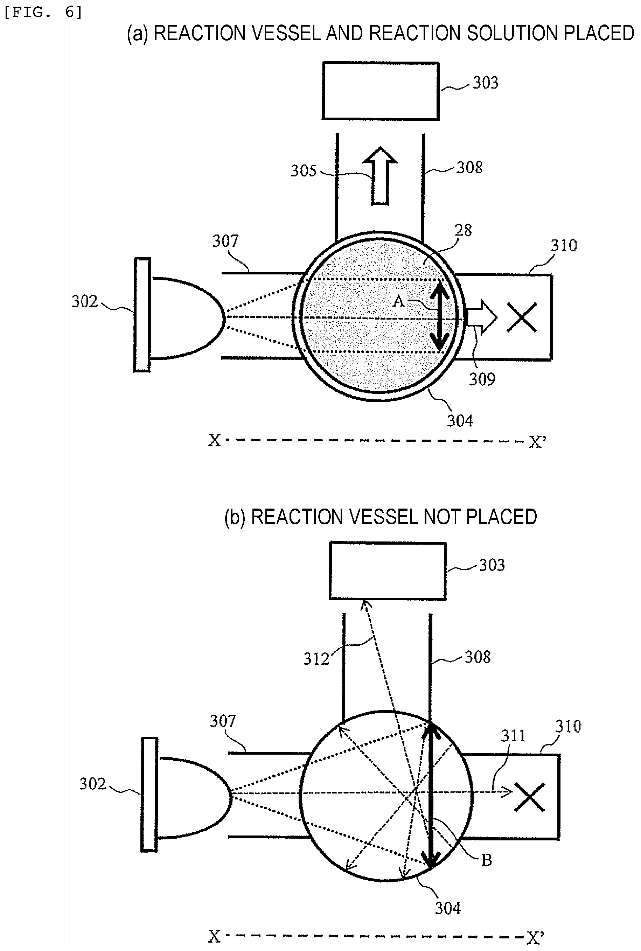

Fig. 6 is a top view illustrating an example of an optical configuration of the blood clotting time detecting unit 21 according to Embodiment 2. The blood clotting time detecting unit 21 according to Embodiment 2 is configured such that a recessed transmitted light absorbing hole 310 is further provided to be opposite to the light source 302 with respect to the analysis port 304 in the blood clotting time detecting unit 21 according to Embodiment 1.

Here, the transmitted light absorbing hole 310 is arranged at a position that is reached by transmitted light 311 of the light irradiated from the light source 302 without colliding with or reflecting from the inner wall of the analysis port 304 when the reaction vessel (for blood clotting analysis) 28 is not arranged in the analysis port 304 as illustrated in (b) of Fig. 6. As in Embodiment 1, the position of the detector 303 is configured such that light 312 of the irradiated light that is reflected by colliding with the inner wall of the analysis port 304 enters into the light receiving slit 308 of the detector 303.

Further, the transmitted light absorbing hole 310 is more than an irradiation range A of light 309 that is irradiated from the light source 302 and transmitted through the reaction vessel 28 containing the reaction solution when the reaction vessel (for blood clotting analysis) 28 is arranged in the analysis port 304 as illustrated in (a) of Fig. 6. In addition, the transmitted light absorbing hole 310 is less than an irradiation range B of light that is irradiated from the light source 302 when the reaction vessel (for blood clotting analysis) 28 is not arranged in the analysis port 304 as illustrated in (b) of Fig. 6.

As a result, in a case where the reaction vessel (for blood clotting analysis) 28 containing the reaction solution is arranged in the analysis port 304 (the case illustrated in (a) of Fig. 6) as in the analysis operation, the light 309 transmitted through the reaction vessel (for blood clotting analysis) 28 enters into the transmitted light absorbing hole 310 and is repeatedly reflected or absorbed on the inner surface of the transmitted light absorbing hole 310 such that the intensity of the light is attenuated. Thus, light that is reflected and returns to the reaction vessel (for blood clotting analysis) 28 side is reduced. Therefore, the amount of scattered light to be measured for analysis can be prevented from being inaccurate.

Further, in a case where the reaction vessel (for blood clotting analysis) 28 is not arranged in the analysis port 304 as illustrated in (b) of Fig. 6, light that is irradiated to the outside of the range of the transmitted light absorbing hole 310 in the irradiation range B of the light collides with and is reflected from the inner wall of the analysis port 304. As a result, the generated reflected light 312 is incident on the detector 303.

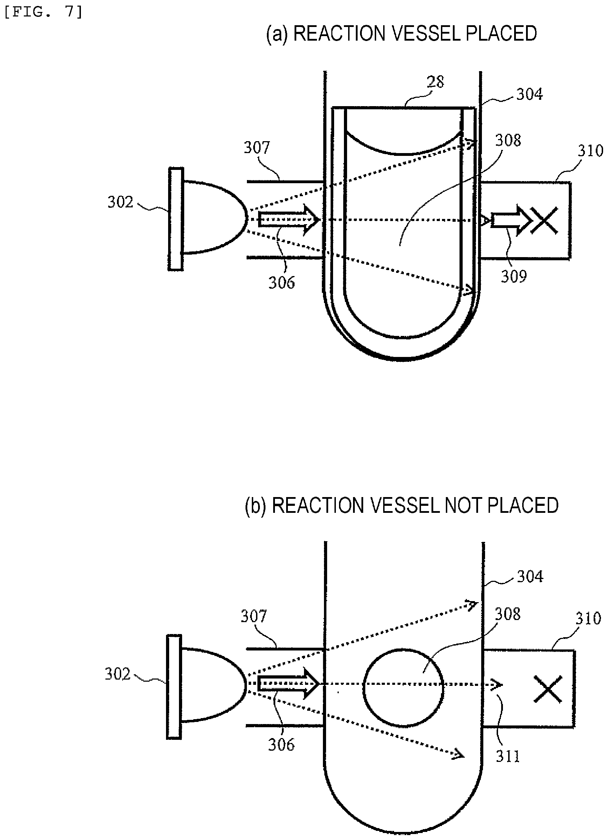

Fig. 7 is a diagram illustrating an optical configuration of the blood clotting time detecting unit 21 according to Embodiment 2 (a cross-sectional view taken from line X-X' of Fig. 6). When the reaction vessel (for blood clotting analysis) 28 is arranged in the analysis port 304 as illustrated in (a) of Fig. 7, the refraction of the light incident on the reaction vessel (for blood clotting analysis) 28 in the vertical direction is small, and the light is reflected from the wall surface of the analysis port 304 during the analysis operation of the blood clotting time. However, in the positional relationship of the arrangement configuration, the light that is reflected from the wall surface of the analysis port 304 is not incident on the detector 303. Therefore, there is little effect on the analysis result obtained from the value of the amount of light detected by the detector 303.

In addition, a part of the light reflected from the wall surface of the analysis port 304 is scattered in the reaction solution stored in the reaction vessel (for blood clotting analysis) 28 such that scattered light is generated. However, in the positional relationship of the arrangement configuration, most of the scattered light is not incident on the detector 303, and thus the influence thereof on the analysis operation of the blood clotting time and the analysis result is negligible.

In other words, the detector 303 does not substantially detect the light generated by the reflection of the light from the wall surface of the analysis port 304. Therefore, light to be detected for the analysis of the blood clotting time, that is, light for estimating a target component in the reaction solution can be accurately and efficiently detected, the reaction solution being the mixed solution of the sample and the reagent stored in the reaction vessel (for blood clotting analysis) 28.

On the other hand, when the reaction vessel (for blood clotting analysis) 28 is not arranged in the analysis port 304 as illustrated in (b) of Fig. 7, even when the light irradiated from the light source 302 is reflected from the wall surface of the analysis port 304 in the vertical direction, the reflected light is not incident on the detector 303 due to the arrangement configuration.

By providing the transmitted light absorbing hole 310 as in the above-described configuration, when photometry is performed in order to check the placement of the reaction vessel (for blood clotting analysis) 28 in a state where the reaction vessel (for blood clotting analysis) 28 is not arranged in the analysis port 304 or in a state where the reaction vessel (for blood clotting analysis) 28 is empty, light generated when the light irradiated from the light source 302 is reflected from the wall surface of the analysis port 304 can be detected by the detector 303. On the other hand, during the analysis operation of the blood clotting time, the detector 303 can accurately detect the amount of scattered light required for the analysis of a target component without detecting the reflected light.

### <Embodiment 8>

In the configuration of Embodiment 2, the light source 302 may be arranged at a position where the irradiated light is substantially parallel in the reaction vessel (for blood clotting analysis) 28 when seen from the top of the reaction vessel (for blood clotting analysis) 28 as illustrated in (a) of Fig. 6, and the width of the irradiation slit 307 and the diameter or maximum diameter of the transmitted light absorbing hole 310 can be configured to be substantially the same as each other (not illustrated). That is, assuming that the reaction vessel (for blood clotting analysis) 28 corresponds to a lens, by arranging the light source 302 at a focal position, light passing through the inside of the reaction vessel (for blood clotting analysis) 28 is parallel.

In this configuration, when the reaction vessel (for blood clotting analysis) 28 is not arranged in the analysis port 304, the irradiation range of the light can be more reliably set to be more than the transmitted light absorbing hole 310. In addition, when the reaction vessel (for blood clotting analysis) 28 containing the reaction solution is arranged in the analysis port 304, the irradiation range of the light can be set to be less than the transmitted light absorbing hole 310. Therefore, the placement of the reaction vessel (for blood clotting analysis) 28 can be accurately checked while reducing the influence on the amount of light to be measured by the detector 303 during the analysis operation of the blood clotting time.

### Reference Signs List

1: automated analyzer
2: blood clotting time analyzing unit
3: biochemical analyzing unit
11: sample disk
12: sample dispensing probe
13: reaction disk
14: second reagent dispensing probe
15: first reagent disk
16: second reagent disk
17: first reagent dispensing probe
18: sample dispensing position
19: photometer
20: blood clotting reagent dispensing probe
21: blood clotting time detecting unit
23: reaction vessel transfer mechanism
24: reaction vessel disposal hole
25: reaction vessel magazine
26: reaction vessel (for biochemical analysis)
27: sample vessel
28: reaction vessel (for blood clotting analysis)
101: interface
102: external output medium
103: display device
104: memory
105: computer
106: printer
107: input device
201: sample dispensing control unit
202: transfer mechanism control unit
203: A/D converter (2)
204: blood clotting reagent dispensing control unit
205: A/D converter (1)
206: reagent dispensing control unit (1)
207: reagent dispensing control unit (2)
302: light source
303: detector
304: analysis port
305: scattered light
306: irradiated light
307: irradiation slit
308: light receiving slit
309: transmitted light
310: transmitted light absorbing hole

## Claims

1. An automated analyzer (1) comprising:
an analysis port (304) in which a reaction vessel (26, 28) can be placed;
a reaction vessel transfer mechanism (23) for transferring the reaction vessel (26, 28) to the analysis port (304);
a light source (302) for irradiating light onto the reaction vessel (26, 28) placed in the analysis port (304);
a detector (303) for detecting some of the light emitted from the light source (302); and
a control unit (202) for controlling the reaction vessel transfer mechanism (23), the light source (302), and the detector (303),
wherein a surface of the analysis port (304) is configured so as to reflect at least some of the light emitted from the light source (302),
the light source (302) and the detector (303) are arranged such that an intensity of the light detected by the detector (303) varies between a second case where the empty reaction vessel (26, 28) is placed in the analysis port (304) and a first case where the reaction vessel (26, 28) is not placed in the analysis port (304) and such that the detector (303) is capable of detecting some of the light reflected from the surface of the analysis port (304),
in the first state before the transfer of the reaction vessel (26, 28) to the analysis port (304) by the reaction vessel transfer mechanism (23), the control unit (202) causes the light source (302) to irradiate light and causes the detector (303) to detect light,in the second state after the transfer of the empty reaction vessel (26, 28) to the analysis port (304) by the reaction vessel transfer mechanism (23), the control unit (202) causes the light source (302) to irradiate light and causes the detector (303) to detect light, and when a difference between an amount of light detected in the first state and an amount of light detected in the second state is less than or equal to a standard value, the control unit produces a warning,
**characterized in that**:
the control unit (202) compares a numerical range of each of three amounts of light having a predetermined width with the detected amount of light, determines whether the state of the analysis port (304) is in the first case, the second case,
or a third case, and produces a warning when the state of the analysis port (304) is different from a predetermined state, the three amounts of light being previously measured and stored and including:
(1) an amount of light that is emitted from the light source (302) and is detected by the detector (303) in the first case where the reaction vessel (26, 28) is not placed in the analysis port (304);
(2) an amount of light that is emitted from the light source (302) and is detected by the detector (303) in the second case where the empty reaction vessel (26, 28) is placed in the analysis port (304); and
(3) an amount of light that is emitted from the light source (302) and is detected by the detector (303) in the third case where the reaction vessel (26, 28) into which a specimen, a diluent, or liquid that does not allow transmission of light is dispensed is placed in the analysis port (304).

2. The automated analyzer (1) according to claim 1,
wherein the surface of the analysis port (304) is configured to reflect at least some of light irradiated from the light source (302) in a state where the reaction vessel (26, 28) into which a specimen or a specimen and a diluent are dispensed is placed, and
when an amount of light that is measured before discharging a reagent into the reaction vessel (26, 28) is less than or equal to an amount of light that is measured after discharging a reagent into the reaction vessel (26, 28), the control unit (202) produces a warning to an operator together with an output of an analysis result.

3. A method of detecting a reaction vessel (26, 28) that is executed by an automated analyzer (1) according to at least one of claims 1 to 2, comprising:
a step of allowing the control unit (202) to cause the light source (302) to irradiate light and to cause the detector (303) to detect light in a first state before the transfer of the reaction vessel (26, 28) to the analysis port (304) by the reaction vessel transfer mechanism (23);a step of allowing the control unit (202) to cause the light source (302) to irradiate light and to cause the detector (303) to detect light in a second state after the transfer of the empty reaction vessel (26, 28) to the analysis port (304) by the reaction vessel transfer mechanism (23); and a step of allowing the control unit (202) to produce a warning when a difference between an amount of light detected in the first state and an amount of light detected in the second state is less than or equal to a standard value,
**characterized by**:
a step of allowing the control unit (202) to compare a numerical range in which each of three amounts of light having a predetermined width with a detected amount of light;
a step of allowing the control unit (202) to determine whether the state of the analysis port (304) is in the first case,
the second case, or a thirdcase,
a step of allowing the control unit (202) to produce a warning when the state of the analysis port (304) is different from a predetermined state, the three amounts of light being previously measured and stored and including:
(1) an amount of light that is emitted from the light source (302) and is detected by the detector (303) in the first case where the reaction vessel (26, 28) is not placed in the analysis port (304);
(2) an amount of light that is emitted from the light source (302) and is detected by the detector (303) in the second case where the empty reaction vessel (26, 28) is placed in the analysis port (304); and
(3) an amount of light that is emitted from the light source (302) and is detected by the detector (303) in the third case where the reaction vessel (26, 28) into which a specimen, a diluent, or liquid that does not allow transmission of light is dispensed is placed in the analysis port (304)

## Patentansprüche

1. Automatisierter Analysator (1), umfassend:
eine Analyseöffnung (304), in der ein Reaktionsgefäß (26, 28) angeordnet werden kann;
einen Reaktionsgefäß-Transfermechanismus (23) zum Transferieren des Reaktionsgefäßes (26, 28) zu der Analyseöffnung (304);
eine Lichtquelle (302) zum Strahlen von Licht auf das Reaktionsgefäß (26, 28), das in der Analyseöffnung (304) angeordnet ist;
einen Detektor (303) zum Detektieren eines Teils des von der Lichtquelle (302) emittierten Lichts; und
eine Steuereinheit (202) zum Steuern des Reaktionsgefäß-Transfermechanismus (23), der Lichtquelle (302) und des Detektors (303),
wobei eine Oberfläche der Analyseöffnung (304) so konfiguriert ist, dass sie zumindest einen Teil des von der Lichtquelle (302) emittierten Lichts reflektiert,
die Lichtquelle (302) und der Detektor (303) so angeordnet sind, dass eine Intensität des von dem Detektor (303) detektierten Lichts zwischen einem zweiten Fall, in dem das leere Reaktionsgefäß (26, 28) in der Analyseöffnung (304) angeordnet ist, und einem ersten Fall, in dem das Reaktionsgefäß (26, 28) nicht in der Analyseöffnung (304) angeordnet ist, und so, dass der Detektor (303) einen Teil des von der Oberfläche der Analyseöffnung (304) reflektierten Lichts detektieren kann,
im ersten Zustand vor dem Transfer des Reaktionsgefäßes (26, 28) zu der Analyseöffnung (304) durch den Reaktionsgefäß-Transfermechanismus (23) die Steuereinheit (202) bewirkt, dass die Lichtquelle (302) Licht ausstrahlt, und bewirkt, dass der Detektor (303) Licht detektiert, im zweiten Zustand nach dem Transfer des leeren Reaktionsgefäßes (26, 28) zu der Analyseöffnung (304) durch den Reaktionsgefäß-Transfermechanismus (23) die Steuereinheit (202) bewirkt, dass die Lichtquelle (302) Licht ausstrahlt, und bewirkt, dass der Detektor (303) Licht detektiert, und wenn eine Differenz zwischen einer im ersten Zustand detektierten Lichtmenge und einer im zweiten Zustand detektierten Lichtmenge kleiner oder gleich einem Standardwert ist, die Steuereinheit eine Warnung erzeugt, **dadurch gekennzeichnet, dass**:
die Steuereinheit (202) einen numerischen Bereich von jeder von drei Lichtmengen mit einer vorbestimmten Breite mit der detektierten Lichtmenge vergleicht, bestimmt, ob der Zustand der Analyseöffnung (304) in dem ersten Fall, dem zweiten Fall oder einem dritten Fall ist, und eine Warnung erzeugt, wenn der Zustand der Analyseöffnung (304) von einem vorbestimmten Zustand verschieden ist, wobei die drei Lichtmengen zuvor gemessen und gespeichert wurden und umfassen:
(1) eine Lichtmenge, die von der Lichtquelle (302) emittiert wird und von dem Detektor (303) im ersten Fall detektiert wird, in dem das Reaktionsgefäß (26, 28) nicht in der Analyseöffnung (304) angeordnet ist;
(2) eine Lichtmenge, die von der Lichtquelle (302) emittiert wird und von dem Detektor (303) im zweiten Fall detektiert wird, in dem das leere Reaktionsgefäß (26, 28) in der Analyseöffnung (304) angeordnet ist; und
(3) eine Lichtmenge, die von der Lichtquelle (302) emittiert wird und von dem Detektor (303) im dritten Fall detektiert wird, in dem das Reaktionsgefäß (26, 28), in das eine Probe, ein Verdünnungsmittel oder eine Flüssigkeit, die keine Lichtübertragung ermöglicht, abgegeben wird, in der Analyseöffnung (304) angeordnet ist.

2. Automatisierter Analysator (1) nach Anspruch 1,
wobei die Oberfläche der Analyseöffnung (304) so konfiguriert ist, dass sie zumindest einen Teil des von der Lichtquelle (302) gestrahlten Lichts in einem Zustand reflektiert, in dem das Reaktionsgefäß (26, 28), in das eine Probe oder eine Probe und ein Verdünnungsmittel abgegeben werden, angeordnet ist, und
wenn eine Lichtmenge, die vor dem Abgeben eines Reagenzes in das Reaktionsgefäß (26, 28) gemessen wird, kleiner oder gleich einer Lichtmenge ist, die nach dem Abgeben eines Reagenzes in das Reaktionsgefäß (26, 28) gemessen wird, die Steuereinheit (202) eine Warnung an einen Bediener zusammen mit einer Ausgabe eines Analyseergebnisses erzeugt.

3. Verfahren zum Detektieren eines Reaktionsgefäßes (26, 28), das von einem automatisierten Analysator (1) nach mindestens einem der Ansprüche 1 bis 2 ausgeführt wird, umfassend:
einen Schritt des Ermöglichens, dass die Steuereinheit (202) bewirkt, dass die Lichtquelle (302) Licht ausstrahlt, und bewirkt, dass der Detektor (303) Licht in einem ersten Zustand vor dem Transfer des Reaktionsgefäßes (26, 28) zu der Analyseöffnung (304) durch den Reaktionsgefäß-Transfermechanismus (23) detektiert; einen Schritt des Ermöglichens, dass die Steuereinheit (202) bewirkt, dass die Lichtquelle (302) Licht ausstrahlt, und bewirkt, dass der Detektor (303) Licht in einem zweiten Zustand nach dem Transfer des leeren Reaktionsgefäßes (26, 28) zu der Analyseöffnung (304) durch den Reaktionsgefäß-Transfermechanismus (23) detektiert; und einen Schritt des Ermöglichens, dass die Steuereinheit (202) eine Warnung erzeugt, wenn eine Differenz zwischen einer im ersten Zustand detektierten Lichtmenge und einer im zweiten Zustand detektierten Lichtmenge kleiner oder gleich einem Standardwert ist,
**gekennzeichnet durch**:
einen Schritt des Ermöglichens, dass die Steuereinheit (202) einen numerischen Bereich, in dem jede von drei Lichtmengen mit einer vorbestimmten Breite mit einer detektierten Lichtmenge verglichen;
einen Schritt des Ermöglichens, dass die Steuereinheit (202) bestimmt, ob der Zustand der Analyseöffnung (304) in dem ersten Fall, dem zweiten Fall oder einem dritten Fall ist,
einen Schritt des Ermöglichens, dass die Steuereinheit (202) eine Warnung erzeugt, wenn der Zustand der Analyseöffnung (304) von einem vorbestimmten Zustand verschieden ist, wobei die drei Lichtmengen zuvor gemessen und gespeichert wurden und umfassen:
(1) eine Lichtmenge, die von der Lichtquelle (302) emittiert wird und von dem Detektor (303) im ersten Fall detektiert wird, in dem das Reaktionsgefäß (26, 28) nicht in der Analyseöffnung (304) angeordnet ist;
(2) eine Lichtmenge, die von der Lichtquelle (302) emittiert wird und von dem Detektor (303) im zweiten Fall detektiert wird, in dem das leere Reaktionsgefäß (26, 28) in der Analyseöffnung (304) angeordnet ist; und
(3) eine Lichtmenge, die von der Lichtquelle (302) emittiert wird und von dem Detektor (303) im dritten Fall detektiert wird, in dem das Reaktionsgefäß (26, 28), in das eine Probe, ein Verdünnungsmittel oder eine Flüssigkeit, die keine Lichtübertragung ermöglicht, abgegeben wird, in der Analyseöffnung (304) angeordnet ist

## Revendications

1. Analyseur automatisé (1) comprenant :
un orifice d'analyse (304) dans lequel un récipient de réaction (26, 28) peut être placé ;
un mécanisme de transfert de récipient de réaction (23) pour transférer le récipient de réaction (26, 28) à l'orifice d'analyse (304) ;
une source de lumière (302) pour irradier de la lumière sur le récipient de réaction (26, 28) placé dans l'orifice d'analyse (304) ;
un détecteur (303) pour détecter une partie de la lumière émise par la source de lumière (302) ; et
une unité de commande (202) pour commander le mécanisme de transfert de récipient de réaction (23), la source de lumière (302) et le détecteur (303),
dans lequel une surface de l'orifice d'analyse (304) est configurée de manière à réfléchir au moins une partie de la lumière émise par la source de lumière (302),
la source de lumière (302) et le détecteur (303) sont agencés de sorte qu'une intensité de la lumière détectée par le détecteur (303) varie entre un deuxième cas où le récipient de réaction vide (26, 28) est placé dans l'orifice d'analyse (304) et un premier cas où le récipient de réaction (26, 28) n'est pas placé dans l'orifice d'analyse (304) et de sorte que le détecteur (303) est capable de détecter une partie de la lumière réfléchie par la surface de l'orifice d'analyse (304),
dans le premier état avant le transfert du récipient de réaction (26, 28) à l'orifice d'analyse (304) par le mécanisme de transfert de récipient de réaction (23), l'unité de commande (202) amène la source de lumière (302) à irradier de la lumière et amène le détecteur (303) à détecter de la lumière, dans le deuxième état après le transfert du récipient de réaction vide (26, 28) à l'orifice d'analyse (304) par le mécanisme de transfert de récipient de réaction (23), l'unité de commande (202) amène la source de lumière (302) à irradier de la lumière et amène le détecteur (303) à détecter de la lumière, et lorsqu'une différence entre une quantité de lumière détectée dans le premier état et une quantité de lumière détectée dans le deuxième état est inférieure ou égale à une valeur standard, l'unité de commande produit un avertissement,
**caractérisé en ce que** :
l'unité de commande (202) compare une plage numérique de chacune de trois quantités de lumière ayant une largeur prédéterminée avec la quantité de lumière détectée, détermine si l'état de l'orifice d'analyse (304) est dans le premier cas, le deuxième cas ou un troisième cas, et produit un avertissement lorsque l'état de l'orifice d'analyse (304) est différent d'un état prédéterminé, les trois quantités de lumière étant précédemment mesurées et stockées et comprenant :
(1) une quantité de lumière qui est émise par la source de lumière (302) et est détectée par le détecteur (303) dans le premier cas où le récipient de réaction (26, 28) n'est pas placé dans l'orifice d'analyse (304) ;
(2) une quantité de lumière qui est émise par la source de lumière (302) et est détectée par le détecteur (303) dans le deuxième cas où le récipient de réaction vide (26, 28) est placé dans l'orifice d'analyse (304) ; et
(3) une quantité de lumière qui est émise par la source de lumière (302) et est détectée par le détecteur (303) dans le troisième cas où le récipient de réaction (26, 28) dans lequel un échantillon, un diluant ou un liquide qui ne permet pas la transmission de lumière est distribué est placé dans l'orifice d'analyse (304).

2. Analyseur automatisé (1) selon la revendication 1,
dans lequel la surface de l'orifice d'analyse (304) est configurée pour réfléchir au moins une partie de la lumière irradiée par la source de lumière (302) dans un état où le récipient de réaction (26, 28) dans lequel un échantillon ou un échantillon et un diluant sont distribués est placé, et
lorsqu'une quantité de lumière qui est mesurée avant de décharger un réactif dans le récipient de réaction (26, 28) est inférieure ou égale à une quantité de lumière qui est mesurée après avoir déchargé un réactif dans le récipient de réaction (26, 28), l'unité de commande (202) produit un avertissement à un opérateur conjointement avec une sortie d'un résultat d'analyse.

3. Procédé de détection d'un récipient de réaction (26, 28) qui est exécuté par un analyseur automatisé (1) selon au moins l'une des revendications 1 à 2, comprenant :
une étape consistant à permettre à l'unité de commande (202) d'amener la source de lumière (302) à irradier de la lumière et d'amener le détecteur (303) à détecter de la lumière dans un premier état avant le transfert du récipient de réaction (26, 28) à l'orifice d'analyse (304) par le mécanisme de transfert de récipient de réaction (23) ; une étape consistant à permettre à l'unité de commande (202) d'amener la source de lumière (302) à irradier de la lumière et d'amener le détecteur (303) à détecter de la lumière dans un deuxième état après le transfert du récipient de réaction vide (26, 28) à l'orifice d'analyse (304) par le mécanisme de transfert de récipient de réaction (23) ; et une étape consistant à permettre à l'unité de commande (202) de produire un avertissement lorsqu'une différence entre une quantité de lumière détectée dans le premier état et une quantité de lumière détectée dans le deuxième état est inférieure ou égale à une valeur standard,
**caractérisé par** :
une étape consistant à permettre à l'unité de commande (202) de comparer une plage numérique dans laquelle chacune de trois quantités de lumière ayant une largeur prédéterminée avec une quantité de lumière détectée ;
une étape consistant à permettre à l'unité de commande (202) de déterminer si l'état de l'orifice d'analyse (304) est dans le premier cas, le deuxième cas ou un troisième cas,
une étape consistant à permettre à l'unité de commande (202) de produire un avertissement lorsque l'état de l'orifice d'analyse (304) est différent d'un état prédéterminé, les trois quantités de lumière étant précédemment mesurées et stockées et comprenant :
(1) une quantité de lumière qui est émise par la source de lumière (302) et est détectée par le détecteur (303) dans le premier cas où le récipient de réaction (26, 28) n'est pas placé dans l'orifice d'analyse (304) ;
(2) une quantité de lumière qui est émise par la source de lumière (302) et est détectée par le détecteur (303) dans le deuxième cas où le récipient de réaction vide (26, 28) est placé dans l'orifice d'analyse (304) ; et
(3) une quantité de lumière qui est émise par la source de lumière (302) et est détectée par le détecteur (303) dans le troisième cas où le récipient de réaction (26, 28) dans lequel un échantillon, un diluant ou un liquide qui ne permet pas la transmission de lumière est distribué est placé dans l'orifice d'analyse (304)
